# EUROPEAN PATENT SPECIFICATION

(11) **EP 2 491 935 B1**
(45) Date of publication and mention of the grant of the patent: **20.04.2016**
(21) Application number: 11305215.3
(22) Date of filing: 28.02.2011
(51) Int. Cl.: A61K 35/50

(54) **Method for kidney transplant**
Verfahren zur Transplantation einer Niere
Procédé de greffe de rein

(43) Date of publication of application: 29.08.2012
(73) Proprietor: Université De Poitiers, 86034 Poitiers Cedex (FR); Centre Hospitalier Universitaire de Poitiers, 86021 Poitiers Cedex (FR)
(72) Inventor: Turhan, Ali, 92410 Ville D'Avray (FR); Hauet, Thierry, 86550 Mignaloux-Beauvoir (FR)
(74) Representative: Flesselles, Bruno F.G.

(56) References cited:
- REINDERS MARLIES E J ET AL: "Multipotent mesenchymal stromal cell therapy in renal disease and kidney transplantation", NEPHROLOGY DIALYSIS TRANSPLANTATION, vol. 25, no. 1, January 2010 (2010-01), pages 17-24, XP002662435, ISSN: 0931-0509
- BENJAMIN D. HUMPHREYS ET AL: "Mesenchymal Stem Cells in Acute Kidney Injury", ANNUAL REVIEW OF MEDICINE, vol. 59, no. 1, 1 February 2008 (2008-02-01), pages 311-325, XP055010708, ISSN: 0066-4219, DOI: 10.1146/annurev.med.59.061506.154239
- HAUSER PETER V ET AL: "Stem Cells Derived from Human Amniotic Fluid Contribute to Acute Kidney Injury Recovery", AMERICAN JOURNAL OF PATHOLOGY, vol. 177, no. 4, October 2010 (2010-10), pages 2011-2021, XP002662436,
- GIRAUD SEBASTIEN ET AL: "Direct Thrombin Inhibitor Prevents Delayed Graft Function in a Porcine Model of Renal Transplantation", TRANSPLANTATION, vol. 87, no. 11, 15 June 2009 (2009-06-15) , pages 1636-1644, XP009152344, WILLIAMS AND WILKINS, BALTIMORE US ISSN: 0041-1337
- CHADE ALEJANDRO R ET AL: "Endothelial Progenitor Cells Restore Renal Function in Chronic Experimental Renovascular Disease", CIRCULATION, vol. 119, no. 4, February 2009 (2009-02), pages 547-557+SUPPL, XP002662437, ISSN: 0009-7322, DOI: 10.1161/CIRCULATIONAHA.108.788653

## Description

The invention relates to the field of organ transplants, and of kidney transplant specifically and provides a new product and method making it possible to improve early primary and therefore the long term graft function.

Stem cell therapy already used in several clinical areas, especially in cardiovascular medicine and hematology needs also to be investigated in case of transplantation particularly in renal transplantation.

Ischemia/reperfusion injury (IRI) is known as one of the central non-immunologic process involved in renal allograft dysfunction which involves vascular and tubular injuries. Indeed, endothelial cells are the first cells subjected to IRI and their activation associated with their necrosis or apoptosis is a main process implicated in extent graft injuries.

Amniotic stem cells are non-tumorigenic multipotent stem cells of of fetal origin and are extracted from amniotic fluid. These cells can be induced to differentiate towards different cells lineages representing each embryonic germ layer including endothelial lineages (De Coppi et al. Nature biotech. 25 1 2007, 100-106).

The regenerative processes after renal ischemia are of crucial interest to investigate in order to improve graft function and to decrease long term dysfunction. Mobilization, homing and transdifferentiation of amniotic stem cells could play an important role in augmenting neovascularisation and endothelial replacement after vascular injury induced by ischemia reperfusion (IR). These cells, in a microenvironment characterized by inflammatory process and oxidative stress; could increase angiogenesis both by stimulating the secretion of angiogenic and vascular growth factors such as Vascular Endothelium Growth Factor (VEGF), Angiopoietin 1 (Ang1), and by providing a new source of progenitor cells that can differentiate into mature vascular endothelial cells.

Despite the promises of stem cell delivery in treating diseases associated with blood vessels dysfunction, the potential of this strategy (use of amniotic stem cells) to protect transplanted kidney has not been explored in large mammal model mimicking clinical transplantation (Chade et al. Circulation 2009 119 547-557).

More than ever, research into the mechanisms of warm ischemia (WI), cold storage, and IR injury (IRI) is needed to maximize the use of the available donor pool and to minimize primary non-function (PNF) and delayed graft function (DGF). Advances in organ preservation solutions and techniques allowed prolongation of hypothermic storage (Gollin et al. Liver transpl. 2008 14 1637-1647).

Ischemia / reperfusion injury is known as one of the central non-immunologic process involved in renal allograft dysfunction leading to major vascular and tubular injuries (Dragun et al. Kidney Int. 2000 58 2166-2177). IR damages favour acute rejection and lays the groundwork for development of chronic graft lesions. Indeed, IR is one of the primordial factors responsible for primary non function and delayed graft function as well as a major factor in long-term graft dysfunction, tissue remodelling and outcome.

The IR syndrome concerns all organs and regroups a large panel of lesional processes responsible for graft injury; particularly important in the case of extended criteria donors and deceased after cardiac death (DCD) donors. These two donor groups are of major interest as they have the potential to increase the donor pool in order to face the current organ shortage. In particular, organs of DCD donors suffer a warm ischemia (after cardiac stop) and the cold ischemia after harvest of the organs and preservation in the cold.

The warm ischemia depends on the conditions of sampling but it is acknowledged that, after 40 minutes of warm ischemia, the kidney has a vasospasm, platelet aggregation and thrombosis.

This point is of particular importance as the average time of arrival of potential DCD donors is 113 ± 31 minutes (Barouk D. et al. Reanimation 2010 (19) 1S: SP 105). The procedure for installing the Fogarty probe alone takes about 20 to 30 minutes. It is then necessary to introduce the two cannulae needed to establish regionalized traffic, which takes about 20-30 minutes more, in the absence of technical difficulties. Thus, from the beginning of the intervention, a minimum time of 40 minutes is required before the organs can be harvested. At this stage of the proceedings, there is an average of 153 minutes from cardiac arrest in the absence of technical difficulties in inserting cannulae.

The cold ischemia is the situation of the kidney between 0°C and 10°C. It may not be stored more than 72 hours. It is known that tubular necrosis increases with the duration of cold ischemia, and that half of the grafts with cold ischemia longer than 36 hours requiring dialysis.

In these conditions, one of the critical targets of IR is the endothelial cells and the microvascular bed (Basile et al. 2007, 72, 151-156, Basile et al. Current opinion in nephrology and hypertension. 2004, 13, 1-7, Cicco et al. Advance in experimental medicine and biology 2005, 566, 363-373). Thus, endothelial cells are the first cells subjected to IRI. Their activation associated with their necrosis or apoptosis is a main process implicated in extent graft injuries.

It was demonstrated that endothelial progenitor cells isolated from peripheral mononuclear cells on experimental renal artery stenosis model in pig (Chade et al. Circulation 2009 119 547-557) were able to preserve microvascular architecture and function and decrease microvascular remodelling in experimental chronic renal artery stenosis, through a single renal artery infusion of autologous endothelial progenitor cells.

Bone Marrow Stem Cells (BMSC) bearing tubular epithelial markers were noticed in female host kidneys following transplantation after provoked renal ischemic injury (Poulsom et al, J Pathol. 2001;195:229-235). Also, BMSC labeled with green fluorescent protein transplanted in sublethally irradiated mouse, with ischemic reperfusion injury 4 weeks afterward, were detected in proximal tubule. These cells comprised 10% of the total cell population of the proximal tubule (Huls et al, Kidney Blood, Press Res. 2008, 31, 104-110 ; Bussolati et al, Contrib Nephrol. 2007, 156, 250-258).

Some studies reported that the main fraction of BMSC that contributes to the regeneration of kidney tissue after acute kidney damage is Mesenchymal Stem Cells (MSC). Their molecular profile shows the expression of CD90, CD73, CD105, CD44, and CD29. They lack the hematopoietic lineage markers such as CD34, CD45, and CD14. MSC are in focus of many researches because they show high multilineage potential. These cells were shown to have the ability to differentiate into bone, fat, cartilage, and muscle cells. MSC have been shown to have protective effects both for chemical (glycerol and cisplatin-induced renal damage) and ischemic/reperfusion kidney damage in rodents (Hopkins et al, J Pathol. 2008, 217, 265-281).

The use of preclinical model in a large animal such as the pig is nevertheless necessary to adapt transplantation processes closely related to the human condition and clarify the mechanisms involved. Inserm U927 unit has a great experience to manage the porcine model of renal transplantation (Favreau et al. Am J Transplant. 2010 10 30-39) and develops a lot of methods to explore the main pathway induced by renal ischemia such as inflammation, oxidative stress, angiogenesis, and fibrosis. Several porcine models have been used to study the mechanisms of WI and the effects of a variety of drugs, and some of them are moving on to clinical evaluation (Faure et al. Biochemical Pharmacology 2003 66 2241-2250, Jayle et al. American J. physiology 2007 292 F1082-1093). Because the anatomical and vascular bed is very similar with human kidneys, these models are very relevant to evaluate the renal response following injury, renal tubular inflammation and the repair processes.

The inventors have shown that regenerative processes can be induced by amniotic fluid stem cell (AFSC) therapy in an *in vivo* model of kidney transplant from a deceased after cardiac arrest donor (renal auto-transplanted pig model).

The invention thus discloses a product containing amniotic stem cells for their use in the treatment of a kidney-transplanted patient.

In the same context, the invention relates to amniotic stem cells for their use in the treatment of a kidney-transplanted patient, *i.e.* for use for improving kidney-graft function of a kidney-transplanted patient.

These amniotic stem cells are of human origin, when used for treating a human patient.

The product of the invention is used post-transplant, together with the usual treatments (such as immuno-suppressors and antibiotics...).

Amniotic stem cells (Amniotic Fluid derived Stem cells, AFS) have been described previously, in particular in De Coppi et al (Nature Biotechnology, 25(1), 2007, pp 100-106). These cells are positive for Class I major histocompatibility (MHC) antigens (HLA-ABC), and some are weakly positive for MHC Class II (HLA-DR). The AFS cells are negative for markers of the hematopoietic lineage (CD45) and of hematopoietic stem cells (CD34, CD133). However, they stain positively for a number of surface markers characteristic of mesenchymal and/or neural stem cells, but not embryonic stem (ES) cells, including CD29, CD44 (hyaluronan receptor), CD73, CD90 and CD105 (endoglin)5,9,10. Human AFS cells also are positive for stage-specific embryonic antigen (SSEA)-4, a marker expressed by ES cells but generally not by adult stem cells. The AFS cells do not express other surface markers characteristic of ES and embryonic germ (EG) cells, SSEA-3 and Tra-1-81. Some lines are weakly positive for Tra-1-60. Over 90% of the cells express the transcription factor Oct4, which has been associated with the maintenance of the undifferentiated state and the pluripotency of ES and EG cells.

In a preferred embodiment, the AFS cells used are HLA-compatible with the patient. This means that the antigens of the MHC complex (class I and II) are identical or prove to be compatible with the good transplant practice. This is preferred to avoid an immune reaction (such as inflammation) around the transplanted kidney which would be detrimental to the success of the graft. Methods for checking the HLA compatibility are well known in the art.

In a preferred embodiment, said cells are injected to the patient between one day and 10 days after kidney transplant, preferably around 6 to 8 days after transplant, in particular 7 days after transplant.

The AFS are preferably injected in the renal artery, and it is preferred to inject between 10⁷ and 10⁸ amniotic stem cells (preferably around 5 x 10⁷ cells), in particular in saline solution.

The cells may be concentrated between 10⁶ and 10⁸ cells per ml, in particular around 10⁷ cells per ml.

As indicated above, in a specific embodiment, said transplanted kidney has suffered a warm ischemia.

In another specific embodiment, said transplanted kidney has suffered a cold ischemia.

In fact, the product according to the invention and use of AFSC are well suited when said transplanted kidney has been harvested on a dead after cardiac arrest (DCD) donor.

The invention also relates to a method of treatment of a patient having received a kidney transplant, comprising the step of injecting amniotic stem cells to said patient after the transplant. The embodiment as mentioned above can apply to said method of treatment.

AFSC can be isolated from amniotic liquid before birth of babies, in particular during caesarean surgery. It is expected that more and more banks of amniotic stem cells will develop in the next future, thus providing a wide access to these cells. Such banks are being set up in Italy, as well as in the US, where in 2009, the first US amniotic stem cell bank was opened in Medford, MA, by Biocell Center, an international company specializing in the cryopreservation and private banking of amniotic fluid stem cells.

It is to be noted that amniotic stem cells may also be used for improving other grafts such as liver, lung, cornea grafts.

### Description of the figure

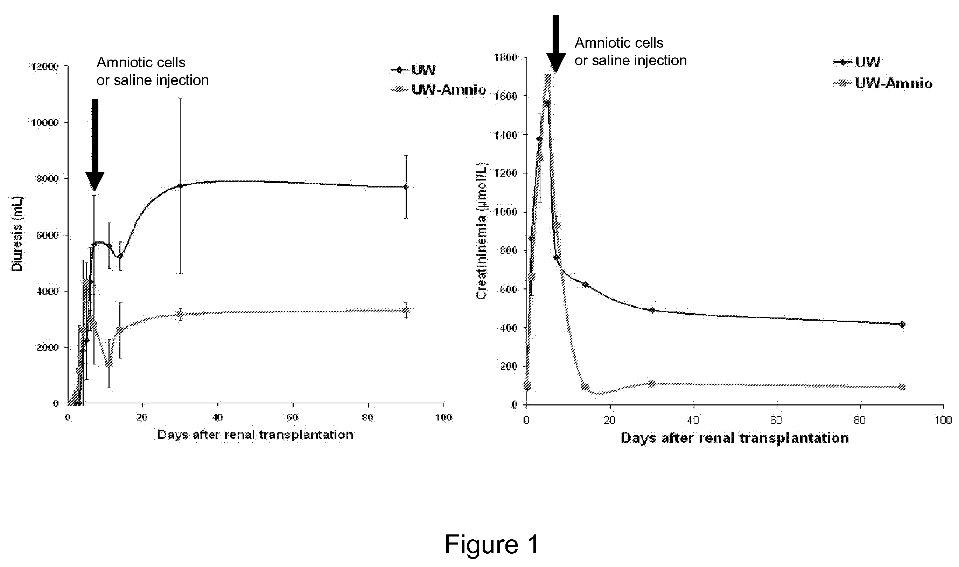

Figure 1 shows creatininemia and diuresis after transplant and injection of either saline (UW) oar saline and amniotic stem cells (UW-Amnio) into the renal artery of kidney transplanted pigs 7 days after transplant. Values are mean ± SD, representing two independent experiments.

### Example

### Preparation of amniotic stem cells

Amniotic liquid is harvested before birth, during the caesarean surgery. Amniotic stem cells are isolated by immunoselection using the markers as indicated above. These cells are grown in appropriate medium for several passages, in the presence of human SCF (stem cell factor) and stored in liquid nitrogen until infusion. Before the infusion, the cells are thawed , washed twice and suspended in saline solution

5x10⁷ cells in 5 ml saline are injected in an autologous model (the pig receives its own cells).

It is to be noted that, for animal experiments, the amniotic stem cells can be labelled with GFP using lentivirus mediated gene transfer, in order to follow the fate of these cells.

### Surgical procedures

A porcine renal auto-transplanted model with controlateral nephrectomy in conditions mimicking DCD donor is used.

Large white male pigs (INRA, GEPA, Le Magneraud, Surgères, France) weighting 30 to 35 kg around 3 months after birth, are prepared as previously described with adapted analgesia during the transplant process and the follow up (Faure et al. Biochemical Pharmacology 2003 66 2241-2250, Jayle et al. American J. physiology 2007 292 F1082-1093).

To mimic conditions found in DCD donors, renal WI (warm ischemia) is induced by clamping the right renal pedicle for 60 min with a vascular non traumatic clamp. These conditions have already been described as a model inducing consistent damage in conditions reproducing DCD (Jayle et al. American J. physiology 2007 292 F1082-1093, Doucet al. American journal of physiology. 2008 295 F179-F191).

Then, the kidney is collected, cold flushed, and preserved for 24 hours in UW solution (University of Wisconsin solution) before transplantation. In each experimental group, the left kidney is removed during transplantation surgery to mimic the nephron mass in transplanted situation. Seven days after grafting, the pigs sustain anesthesia and a laparotomy to isolate renal artery.

The renal artery is clamped with vascular non traumatic clamp and injected slowly during 2 minutes with 5ml of saline solution containing either amniotic cells or only saline, and the clamp is released.

Three groups of six animals can be studied:
1) UW: Pigs' kidneys preserved in University of Wisconsin (UW) solution and and intra renal artery injection of saline solution, 7 days after kidney graft.
2) UW-Amnio: Pigs' kidneys preserved in University of Wisconsin (UW) solution and intra renal artery injection of saline solution with amniotic stem cells 7 days after kidney graft. It's auto-cell therapy; pig previously collected at the birth will receive its own cells.
3) Normal animals: age, and weight mass matched group.

Pigs are followed for 3 months and sacrificed.

Renal tissue samples and other tissue such as heart, liver, lung are collected for *in vitro* studies.

Pigs are placed in a metabolic cage before and 1, 3, 7, 14, 30 and 90 days after transplantation to allow blood and specific 24 h urine collections.

Plasma creatinine and urinary proteins are measured using an automatic analyzer (Modular automatic analyzer, Roche Diagnostic, Meylan, France). These data, coupled with diuresis, make it possible to calculate GFR.

### Biological analysis

GFP expression is investigated by RT-PCR technique in renal and other tissue sample, and circulated cells to check homing of amniotic cells. In kidneys, it is believed that GFP positive cells will localize with endothelial, distal and proximal tubular cells markers respectively KDR or CD31, cytokeratin 7 and CD10 using immunohistochemistry.

To characterize endothelium injury and effect of amniotic stem cells, endothelial cells activation is investigated by expression of VCAM-1, MCP-1 by western blot or RT-PCR in renal tissue sample. Interleukin such as IL10, potentially released by amniotic stem cells in the blood is measured during the early follow up. In addition, growth factor such as VEGF is assessed in the same blood sample. Angiogenesis in graft is characterized by testing angiogenic factors expression like VEGF, Ang-1, Flt-1 (VEGF Receptor 1) and Flk-1 (VEGF Receptor 2) by western blot.

HIF1a pathway is investigated by measuring HIF1a expression in renal tissue by western blot.

Micro-vascularisation is studied using microCT analyser. Briefly, an intravascular radio-opaque silicone polymer (Microfil MV122; Flow Tech, Carver, MA, USA) is injected in renal artery after sacrifice until the polymer drains freely from the segmental vein. A lobe of the polymer-filled tissue is subsequently trimmed, prepared, and scanned using a micro-computed tomography scanner, as described previously (Favreau et al. Kidney Int. 2010, 78:1110-1118). It is possible to study spatial density, average diameter, and tortuousity of cortical microvessels supporting potential neo-vascularisation.

The main issue inducing graft lost is renal fibrosis associated to vascular remodelling. Fibrosis is assessed by red Sirius staining in graft and expression of pro-fibrotic factors such as TGF beta and its effector Smad pathway and CTGF, and PAI-1 associated to metalloproteinase expression such as MMP-2.

### Results

The preliminary results obtained from two pigs in conditions as described above show that injection of amniotic cell in renal artery 7 days after transplantation as an autologous transplant leads to 100% survival compared to 30% survival in pig without amniotic cells in this model that combines warm and cold ischemia.

In addition, graft function recovery was improved in UW-Amnio group as shown in Figure 1 with normalization of creatininemia and diuresis at 14 days. Fibrosis development at 3 months post-transplantation was reduced compared with UW group.

## Claims

1. Amniotic stem cells for use for improving kidney-graft function of a kidney-transplanted patient.

2. Amniotic stem cells for use in the therapy of a kidney-transplanted patient according to claim 1, wherein said cells are HLA-compatible with the patient.

3. Amniotic stem cells for use in the therapy of a kidney-transplanted patient according to any of claims 1 to 2, containing between 10⁷ and 10⁸ amniotic stem cells.

4. Amniotic stem cells for use in the therapy of a kidney-transplanted patient according to any of claims 1 to 3, wherein said stem cells are provided in saline solution.

5. Amniotic stem cells for use in the therapy of a kidney-transplanted patient according to any of claims 1 to 4, wherein the transplanted kidney has suffered a warm ischemia.

6. Amniotic cells for use in the therapy of a kidney-transplanted patient of any according to claims 1 to 4, wherein the transplanted kidney has suffered a cold ischemia.

7. Amniotic stem cells for use in the therapy of a kidney-transplanted patient according to any of claims 1 to 6, wherein the transplanted kidney has been harvested on a dead after cardiac arrest (DCD) donor.

## Patentansprüche

1. Amniotische Stammzellen für die Verwendung zur Verbesserung der Nierentransplantat-Funktion bei einem nierentransplantierten Patienten.

2. Amniotische Stammzellen für die Verwendung bei der Therapie eines nierentransplantierten Patienten nach Anspruch 1, wobei die Zellen mit dem Patienten in Bezug auf die HLA kompatibel sind.

3. Amniotische Stammzellen für die Verwendung bei der Therapie eines nierentransplantierten Patienten nach einem der Ansprüche 1 bis 2, enthaltend zwischen 10⁷ und 10⁸ amniotische Stammzellen.

4. Amniotische Stammzellen für die Verwendung bei der Therapie eines nierentransplantierten Patienten nach einem der Ansprüche 1 bis 3, wobei die Stammzellen in Kochsalzlösung bereitgestellt werden.

5. Amniotische Stammzellen für die Verwendung bei der Therapie eines nierentransplantierten Patienten nach einem der Ansprüche 1 bis 4, wobei die transplantierte Niere eine warme Ischämie erlitten hat.

6. Amniotische Stammzellen für die Verwendung bei der Therapie eines nierentransplantierten Patienten nach einem der Ansprüche 1 bis 4, wobei die transplantierte Niere eine kalte Ischämie erlitten hat.

7. Amniotische Stammzellen für die Verwendung bei der Therapie eines nierentransplantierten Patienten nach einem der Ansprüche 1 bis 6, wobei die transplantierte Niere einem Spender nach dem Tod durch Herzstillstand (dead after cardiac arrest, DCD) entnommen wird.

## Revendications

1. Cellules souches amniotiques pour leur utilisation pour améliorer la fonction d'une greffe de rein chez un patient transplanté rénal.

2. Cellules souches amniotiques pour leur utilisation dans la thérapie d'un patient transplanté rénal selon la revendication 1, lesdites cellules étant HLA-compatibles avec le patient.

3. Cellules souches amniotiques pour leur utilisation dans la thérapie d'un patient transplanté rénal selon l'une quelconque des revendications 1 à 2, contenant entre 10⁷ et 10⁸ cellules souches amniotiques.

4. Cellules souches amniotiques pour leur utilisation dans la thérapie d'un patient transplanté rénal selon l'une quelconque des revendications 1 à 3, lesdites cellules souches étant fournies en solution saline.

5. Cellules souches amniotiques pour leur utilisation dans la thérapie d'un patient transplanté rénal selon l'une quelconque des revendications 1 à 4, où le rein transplanté a souffert d'une ischémie à chaud.

6. Cellules souches amniotiques pour leur utilisation dans la thérapie d'un patient transplanté rénal selon l'une quelconque des revendications 1 à 4, où le rein transplanté a souffert d'une ischémie à froid.

7. Cellules souches amniotiques leur pour utilisation dans la thérapie d'un patient transplanté rénal selon l'une quelconque des revendications 1 à 6, le rein transplanté ayant été récolté sur un donneur décédé après un arrêt cardiaque (DCD).
